# EUROPEAN PATENT SPECIFICATION

(11) **EP 1 438 026 B1**
(45) Date of publication and mention of the grant of the patent: **13.04.2011**
(21) Application number: 02802169.9
(22) Date of filing: 22.10.2002
(51) Int. Cl.: A61K 9/10, A61K 47/10, A61K 47/32

(54) **TASTE MASKING SPILL-RESISTANT FORMULATION**
DEN GESCHMACK VERDECKENDE AUSLAUFSICHERE FORMULIERUNG
DISSIMULATION DU GOUT DE FORMULATION RESISTANTE A L'ECOULEMENT

(30) Priority: 22.10.2001 US 330447 P
(43) Date of publication of application: 21.07.2004
(73) Proprietor: TARO PHARMACEUTICAL INDUSTRIES LIMITED, 26110 Haifa (IL)
(72) Inventor: MOROS, Daniel, A., Larchmont, NY 10538-4145 (US); GAO, Shen, Brampton, Ontario L6P 1B6 (CA); MOLDENHAUER, Maxine, G., Acton, Ontario L7J 2L7 (CA)
(74) Representative: Chajmowicz, Marion
(86) International application number: PCT/US2002/033478
(87) International publication number: WO 2003/034991

(56) References cited:
- EP-A- 0 614 659
- EP-A- 0 815 854
- US-A- 4 427 681
- US-A- 4 806 354
- US-A- 5 288 479
- US-A- 5 300 302
- US-A- 5 602 182
- US-A- 5 616 621
- US-A- 6 010 683
- US-A- 6 071 523
- US-A- 6 102 254

## Description

### BACKGROUND OF THE INVENTION

The invention relates to a taste masking spill resistant pharmaceutical composition, comprising an elegant spill resistant formulation with taste masking concentrations of low-molecular weight polyethylene glycol (PEG). This composition is less bitter, sweeter and has better overall flavor than current pharmaceutical compositions, while maintaining advantageous spill resistant properties.

Heretofore, pharmaceutical agents for systemic treatment by oral administration have generally been formulated in solid form as pills or capsules or in liquid form. Children, the elderly and people with motor problems often have problems swallowing pills and capsules. It is also difficult to administer medicine in liquid form to children, even when the liquid has been thickened to a syrup, and the elderly and those with motor problems also have difficulty with the self administration of liquid, especially when it is necessary to measure a specific unit dose.

Important requirements for any non-solid pharmaceutical formulation for oral administration include palatability to children and adults, stability, i.e. a long shelf life, compatibility of formulation components with active agent, and accuracy of administration of the required dose. Ease of administration is a long-standing need for such formulations.

Pharmaceutical preparations in semisolid form for topical application are well known in the art. Such preparations include gels, pastes, creams and ointments for use on the skin, teeth and mucous membranes. Antacids and anti-ulcer agents in suspension and gel form for coating the mucous lining of the stomach are also well known in the art.

References have been made to incorporating pharmaceutical agents into thickened vehicles for oral administration, as for example those disclosed in U.S. Pat. Nos. Wiczer, US 4,305,933, Ravel et al., 4,576,645, Munshi US 4,427,681, and Tachon, US 5,300,302, and Gorman et al., US 5,288,479. There is a need for spill resistant pharmaceutical compositions that do not contain gelatin, pectin, or seaweed polysaccharides such as agar, algin, carrageenan or furcelleran, with commercially acceptable taste.

However, these vehicles all suffer from one or more disadvantages, such as the presence of a component which is undesirable for administration to children and/or is incompatible with many pharmaceutical agents, the presence of an emulsion which is difficult to manufacture and tends to be unstable and/or inadequate viscosity, or bitter taste.

There is a need for an economical formulation of systemic pharmaceutical agents in easily-administered form, as well as a need for easily administered spill resistant pharmaceutical formulations which can be measured and administered effortlessly to children and by adults with motor problems. Such a formulation must be palatable, ie. not bitter, with an acceptable overall flavor and texture.

Spill resistant pharmaceutical formulations for oral administration are described in U.S. Patent 6,071,523 issued to Mehta et al., and U.S. Patent 6,102,254 issued to Ross. There remains a need for formulations with improved palatability, and a need for balancing the components of the formulation to achieve this goal while maintaining the other requisite characteristics of a spill resistant formulation.

### SUMMARY OF THE INVENTION

The invention relates to a taste masking spill resistant pharmaceutical composition, comprising an elegant spill resistant formulation with taste masking concentrations of polyethylene glycol (PEG) from at least about 1% to about 25%. Specific examples include PEG concentrations of about 5, 10, 15, and below 20%. The composition can be formulated as a placebo (base) or can further comprise an active ingredient. This composition is less bitter, sweeter and has better overall flavor than current pharmaceutical compositions, while maintaining advantageous spill resistant properties. The formulations contain orally active therapeutic agents in semisolid form for oral administration, in a multidose container or a single dose device.

The invention provides pharmaceutical agents useful for systemic treatment by the oral route in a form which is convenient to administer to children, which is convenient for self administration of aging adults, as well as adults with motor problems, with improved taste.

The invention provides pharmaceutical agents useful for systemic treatment by oral administration in a composition which is provided in a device from which it is particularly easy to administer and convenient to measure single dosage units of the composition, and avoids the problems of liquid formulations, such as spillage.

### DESCRIPTION OF THE DRAWINGS

Figure 1 shows the solubility of acetaminophen in water, glycerin and propylene glycol.

Figure 2 shows a viscosity - carbomer concentration relationship.

### DETAILED DESCRIPTION

In describing embodiments of the present invention, specific terminology is employed for the sake of clarity. However, the invention is not intended to be limited to the specific terminology so selected. It is to be understood that each specific element includes all technical equivalents, which operate in a similar manner to accomplish a similar purpose. The above-described embodiments of the invention may be modified or varied, and elements added or omitted, without departing from the invention, as appreciated by those skilled in the art in light of the above teachings. Each reference cited here is incorporated by reference as if each were individually incorporated by reference.

Where the term "pharmaceutical" is used herein, it should be understood to include prescription, over the counter, GRAS (generally recognized as safe), nutraceutical, and other products whether subject to approval by a drug regulatory agency or not. The pharmaceutical agents useful in the inventive formulations are typically bitter.

The term "spill resistant formulation" refers here to a product which, as sold, has viscosity in a certain range (e.g. 5,000 to 20,000 cps), is a semi-solid, is easy to administer accurately, has spill-resistant consistency, is storage stable, and has mutually compatible ingredients, as described in Mehta et al., US 6,071,523. Viscosity is measured using a Brookfield Viscometer with a 'C' spindle with Helipath movement at 20 RPM and 20-25 degrees C, or equivalent. Viscosity decreases slightly with increasing temperature.

Semi-solid character in this context generally indicates a formulation that has a viscometric yield value determined as a relative value, e.g. using a Brookfield Viscometer to measure a shear vs. stress curve. Ease of administration is intended to mean (a) extrudability under light manual pressure from a squeezable container or a proxy (e.g. a syringe with a 5 mm orifice), and (b) spreadability in a spoon bowl measured by extruding the formulation into a spoon bowl and determining whether the material levels or spreads to the edges of the spoon bowl. Spreadability also contributes to accuracy of measurement.

A spill-resistant formulation according to the invention begins to spill from a spoon bowl during test periods of vibrations, inversion, and tilting, but slowly enough to conform with practical time limits between dispensing and ingesting, and quickly enough to enable the product to be readily consumed from a spoon bowl by a patient.

Mutual compatibility of the components means that they do not separate in preparation and storage for the equivalent of two years at room temperature (as indicated by three-months accelerated stability testing at 40 degrees centigrade and 75% relative humidity). Storage stability means that the materials do not lose their desirable properties during storage for the same period. Preferred compositions do not exhibit a drop in viscosity of more than 50% or an increase in viscosity of more than 100% during that period.

The inventive formulations have attractive appearance, suitable texture and mouthfeel. The components are mutually compatible in that they do not interfere with the bioactivity of the pharmaceutical agent or physical properties of the vehicle, and the components do not separate and retain their properties.

The invention relates to a palatable spill resistant pharmaceutical composition, comprising an elegant spill resistant base with taste masking concentrations of polyethylene glycol (PEG) from at least about 1% to about 20%, from about 5% to about 15%, about 5% to about 10%, or about 10% to about 15%. Specific examples include PEG concentrations of about 5%, about 10% and about 15%. The PEG may include PEG 400, PEG 600, PEG 1000, or combinations. The PEG component may be selected depending on the other components. Higher values for PEG are less toxic, as are concentrations of 15% or less. Toxicology studies are required for a formulation with higher than 15% PEG. The composition can further comprise an active ingredient. The taste-masking PEG component is a low-weight PEG. Useful low-weight PEGs include PEG 200, 300 400, 540, 600, 800, 900, 1000, 1450, 1540 and 2000, and combinations. These low-weight PEGs can be used to replace other liquids and solvents for bitter active agent suspensions and solutions.

The formulation may comprise about 5% to about 15% PEG, up to about 30 or 45 % water, up to about 50% glycerin, 50% active ingredient, up to about 0.3% to about 0.4% sucralose liquid concentrate, about 0.5 to about 0.6 % Carbomer 934P, and a flavor selected from grape, cherry, or bubble gum.

In one embodiment the composition comprises from about 1% to about 20% polyethylene glycol (PEG), up to about 40 % water, up to about 50% active ingredient, up to about .4% sucralose liquid concentrate, up to about 0.59% Carbomer 934P, up to about 0.15% grape flavor, and up to about 0.4% of a taste masking agent.

Other embodiments include the composition comprising about 30% water, about 50% glycerin, about 15% polyethylene glycol 1000, about 0.4% sucralose liquid concentrate, about 0.29% Carbomer 934P, about 0.15% grape flavor, and about 0.4% masking agent.

Another embodiment includes the composition comprising about 38.5% water, about 50.0% glycerin, about 10.0% polyethylene glycol 1000, about 0.55% Carbomer 934P, about 0.15% grape bubble gum flavor, and about 0.30% sucralose liquid concentrate.

Yet another embodiment includes a composition comprising about 33.3% water, about 50.0% glycerin, about 15.0% polyethylene glycol 1000, about 0.59% Carbomer 934P, about 0.15% grape bubble gum flavor, and about 0.30% sucralose liquid concentrate.

A composition comprises about 43.3% water, about 50.0% glycerin, about 5.0% polyethylene glycol 1000, about 0.59% Carbomer 934P, about 0.15% grape bubble gum flavor, and about 0.30% sucralose liquid concentrate.

In contrast to prior formulations e.g. Gorman et al., US 5,288,479, the spill resistant pharmaceutical composition does not contain a seaweed polysaccharide such as agar, algin, carrageenan or furcelleran, and does not include gelatin or pectin, or gums like xanthan gum. Such seaweed polysaccharides gelatin agents and gums are incompatible with the desired characteristics of the inventive spill-resistant formulations, which derive desirable rheological properties from the use of carboxyvinyl polymers and similar components, such as high-weight PEGs and cellulose derivatives.

The pharmaceutical compositions of the invention comprise a pharmaceutical agent in an effective amount for systemic treatment by oral administration in admixture with a pharmaceutically acceptable vehicle comprising a thickening agent in an amount which provides a semisolid, such as a gel or a paste suspension. The semisolid has a Brookfield viscosity at of above 2500 cps, preferably 2500 to 70,000 cps more preferably 3500-25,000 cps, about 5000-10,000 cps, about 5000-15,000 cps, about 5000-20,000 cps, about 6000-17,000, or about 8,000 to about 11,000 cps. In the present application, viscosity refers to Brookfield viscosity, measured at 25°C and a spindle speed of 10 rpm, unless otherwise noted, which measures viscosity of plastic materials.

In general, the viscosity of the compositions of the invention can be varied by the choice and amount of thickening agent and other components to a consistency which permits the composition to be readily squeezed and flow through a relatively narrow orifice, i.e. of the order of about 1 to 10 mm in diameter.

By systemic treatment is meant treatment that affects the body as a whole, as compared to topical treatment, which affects only that part of the body to which it is applied, i.e. skin, teeth or particular mucous membrane, such as the lining of the stomach.

Orally active pharmaceutical agents which may be present in the semisolid compositions of the invention are those useful for systemic treatment by oral administration. The advantageous properties of the formulation are most apparent when the pharmaceutical agent tastes bitter in the absence of a taste masking component. Alternatively, a non-active component of the formulation such as propylene glycol is bitter or both the pharmaceutical agent and the base taste bitter in the absence of taste masking. The pharmaceutical agent may be an analgesic, non-steroidal anti-inflammatory, antihistamine, cough suppressant, expectorant, bronchodilator, anti-infective, CNS active drug, cardiovascular drug, antineoplastic, cholesterol-lowering drug, anti-emetic, vitamin, mineral supplement and fecal softener. The pharmaceutical agent may be selected from the group consisting of acetaminophen, aspirin, ibuprofen, diphenhydramine, dextromethorphan, guaifenesin, pseudoephedrine, carbidopa, levodopa, terfenadine, ranitidine, ciprofloxacin, triazolam, fluconazole, propranolol, acyclovir, fluoxetine, enalapril, diltiazem, lovastatin and a pharmaceutically acceptable salt or ester thereof.

Particular examples of therapeutic agents include bitter agents selected from:

analgesics, such as acetaminophen, codeine, aspirin and dihydrocodeinone;

anti-inflammatory agents, such as ibuprofen, naproxen and diclofenac;

anti-histamines including H₁ -blockers, such as chlorpheniramine, terfenadine, loratidine, astemizole and cetirizine and H₂ -blockers, such as cimetidine and ranitidine;

anti-infectives including: antibacterials such as sulfa drugs, i.e. sulfisoxazole, and cephalosporins, penicillins, and macrolide antibiotics;

quinolones, i.e. ciprofloxacin and ofloxacin;

tetracyclines, i.e. tetracycline;

anti-virals, i.e acyclovir and amantadine and anti-fungals, i.e. fluconozole;

bronchodilators, such as albuterol, metaproterenol and theophylline;

cough suppressants, such as dextromethorphan;

expectorants, such as guaifenesin;

CNS active agents, including: hypnotics, such as triazolam; sedatives, such as phenobarbital;

tranquilizers, such as chlorpromazine and diazepam; antidepressants, such as fluoxetine and nortriptylline;

anti-convulsants, such as carbamazepine and ethosuximide and anti-Parkinson's agents, such as L-DOPA;

cardiovascular drugs including: diuretics, such as hydrochlorthiazide;

anti-hypertensives including: beta-blockers, such as propranolol;

ACE inhibitors, such as captopril and enalapril; calcium channel blockers, such as diltiazem;

anti-anginals, same as anti-hypertensive agents;

cardiac glycosides, such as digoxin;

antineoplastics, such as 5-fluorouracil and cyclophosphamide;

cholesterol-lowering agents such as lovastatin;

anti-emetics, such as metoclopramide;

vitamins;

minerals, such as iron, calcium and zinc salts and fecal softeners, such as docusate;

plant extracts, such as echinacea, gingko biloba, St. John's wort, etc.

Useful pharmaceutical agents of course include pharmaceutically acceptable salts and esters of the named compositions.

The semisolid compositions of the invention have a liquid base, which is a palatable pharmaceutically acceptable solvent, which may dissolve or suspend the active pharmaceutical agent. Solvents include water, propylene glycol, glycerin and mixtures thereof. In some instances it may be necessary to include a compound which is effective to solubilize the active pharmaceutical agent in the solvent; for example, lactic acid is used in an aqueous formulation of ciprofloxacin hydrochloride to solubilize this active ingredient.

According to the invention, a pharmaceutically acceptable compatible semi-solid thickening agent is used in the compositions of the invention, providing of course that the thickening agent is compatible with the active agent, the solvent base, and other components. Useful thickening agents include water soluble carboxyvinyl polymers, such as those sold under the names of carbomer and Carbopol™, which is produced by B. F. Goodrich Chemical Group.

A sweetener may be added to the composition of the invention in an amount necessary to increase sweetness.

Ingredients such as flavoring, coloring matter, filler, preservative, buffer, sodium chloride and carriers usual in pharmaceutical compositions can also be present in the semisolid compositions of the invention.

The following examples further illustrate the invention, but must not be construed as limiting the invention in any manner.

### EXAMPLE 1

A semi-solid gel product for oral administration was designed with spill resistant characteristics (vibration resistance and invertability) and an easy spreadability to obtain an accurate measured dosage.

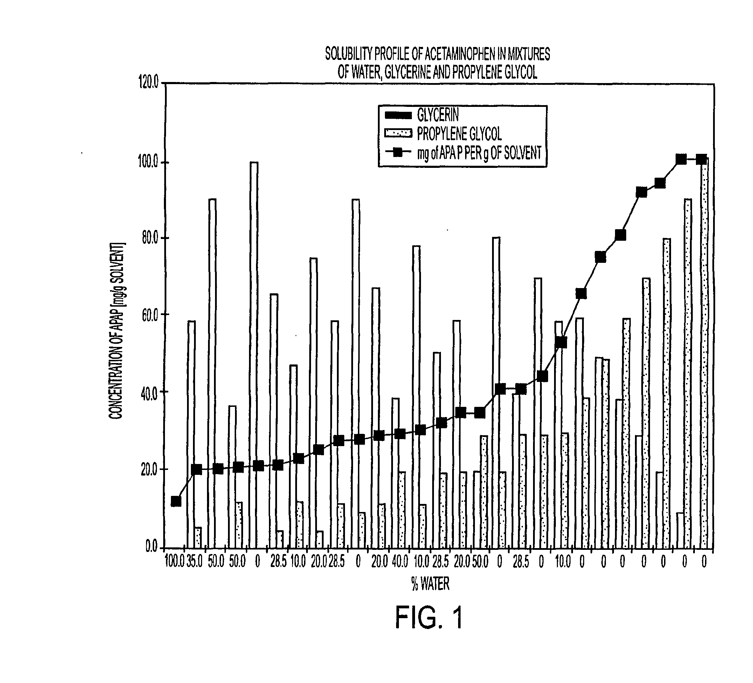

Acetaminophen was supplied as a white powdery material. It is very slightly soluble in water (12.9mg/g), slightly soluble in glycerin (22.2mg/g) and soluble in propylene glycol (101.2mg/g) (Figure 1). Acetaminophen is also soluble in methanol, ethanol dimethylformamide, ethylene dichloride, acetone and ethyl acetate. (Merck Index 12th Ed.)

Acetaminophen has a chemical formula of C₈H₉NO₂ and a molecular weight of 151.16 (USP 24 and Merck Index 12th Ed.). The chemical name is N-(4-hydroxyphenyl)acetamide or 4' - Hydroxyacetanilide.

A semi-solid base was made having a liquid base comprised of glycerin, propylene glycol and water. Propylene glycol has become widely used as a solvent, extractant, and preservative in a variety of pharmaceutical formulations (Handbook of Pharm. Excipients). As an antiseptic it is similar to ethanol, and against molds it is similar to glycerin and only slightly less effective than ethanol.

The IIG range of propylene glycol is 2.0-50.0% for oral solution and 0.69-70% for oral suspension. The level of 25% w/w propylene glycol was chosen for its solvent effect. It was found that the propylene glycol imparts a bitter taste to the gel and so an alternative was required.

Glycerin is also used in a wide variety of pharmaceutical formulations. In oral solution glycerin is used as a solvent, sweetener agent, antimicrobial preservatives, and viscosity-increasing agent. The IIG range of glycerin 5.0-50.0% for oral syrup.

The levels of 40% and 50% w/w glycerol with 25% w/w propylene glycol were used for acetaminophen solubility study. It was found that the batches remained as a solution with glycerin amount at both 40% and 50% w/w. A six cycles of freeze and thaw study was conducted, and no crystallization was found. Therefore, 40% glycerin was chosen as the final concentration if 25% of propylene glycol was added to the formula.

### Polyethylene Glycol

Polyethylene glycols are stable, hydrophilic substances that can be used to enhance the aqueous solubility or dissolution characteristics of poor soluble compounds (Handbook of Pharm. Excipients, incorporated herein by reference). Polyethylene glycols do not support microbial growth (Handbook of Pharm. Excipients). Both polyethylene glycol 600 and polyethylene glycol 1000 were evaluated for the formula. Both of them can be used to replace propylene glycol as a solvent for acetaminophen. More than 20 cycles of freeze - thaw study was performed to a acetaminophen NSG batch contained 15% of polyethylene glycol 1000 and 40% glycerin, and no crystal was found in the sample indicating a stable solution was formed.

Taste improvement was observed after introducing polyethylene glycol into the formula. There is no bitter taste detectable after replacing the propylene glycol with the polyethylene glycol.

A final formula contains 15% of polyethylene glycol 1000 and 50% glycerin.

### Carbomer

Several thickening agents can be used in the formulation including sodium carboxymethylcellulose, polyethylene glycol, hydroxypropyl methylcellulose, hydroxy propyl cellulose, microcrystalline cellulose and carbomer (carboxyvinyl polymer). Certain polyethylene glycols with higher molecular weight (>1540, e.g., PEG 2000, 3000, 3350, 4000, 4600 and 8000) may not perform well as bitter masking agents, but instead may be useful as thickeners. Carbomer was chosen for this formulation due to its clarity and rheological properties, specifically high yield value and shear thinning quality.

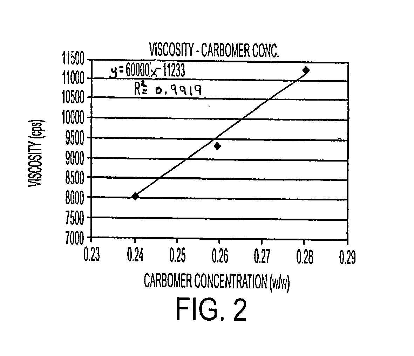

In this batch, a level of 0.265% w/w carbomer (Figure 2) provided a viscosity within the target range of 6,000 to 17,000 cps (as measured using a Brookfield Viscometer).

Carbomer based gels derive their viscosity by pH adjustment and the viscosity of this product is integral to the non-spill properties. Two 3.0-kg batches of Acetaminophen NonSpil™ Gel (Taro) were subdivided into portions and adjusted to different pH values to conduct the viscosity study. The plateau region occurs from about pH 6.4 to 7.0. The gels should have a pH of 6.4 or greater to ensure the viscosity will be higher than 8000 cps and will exhibit non-spill properties. The pH should not exceed approximately 7.0 as the viscosity starts to decrease.

### EXAMPLE 2

### Acetaminophen Suspension NSG Formulation Development

### Introduction

It was found that the acetaminophen solution NSG had a somewhat bitter after taste. A suspension version of acetaminophen NSG was developed to give a better taste. The suspension may also offer greater chemical stability of acetaminophen since the drug is not in solution form. Three flavor versions of acetaminophen NSG suspension were developed: cherry, grape and bubble gum.

### Propylene Glycol

Propylene glycol was decreased to 0.5% in order to decrease the solubility of acetaminophen

### Glycerin

Glycerin was eliminated from the formula in order to decrease the solubility of acetaminophen

### Poloxamer 188

Poloxamer 188 at a level of 0.05% w/w was used to provide the desired effects of retarding crystal growth. The usual concentration of surfactants varies from 0.05% to 0.5% and depends on the solids content intended for suspension. On the other hand, surfactants at concentration less than about 0.05% can result in incomplete wetting. Concentration greater than 0.5% surfactant may solubilize ultrafine particles and lead eventually to change the particle size distribution and crystal growth. (Pharm. Dosage forms: disperse system)

### Acetaminophen NSG Suspension Carbomer Concentration Investigation

According to the specific gravity, the concentration of acetaminophen in the batch would be changed from 2.8% to 2.89%. Therefore, the carbomer concentration would be adjusted to obtain the optimal viscosity.

The following two batches were made for carbomer concentration investigation:

**TABLE 1**

| **Lot No.** | **Size** | **Carbomer** | **Viscosity** |
|---|---|---|---|
| S175-52435 | 1.0 kg | 0.30% | |
| S175-52436 | 1.0 kg | 0.28% | |

| | | | |
|---|---|---|---|
| Conclusion: The final concentration of carbomer is 0.30%. | | | |

### Masking Agent Concentration Investigation

Studies were performed to investigate the masking agent of the formula (a blend from Bell Flavors). Conclusion: The masking agent is optional and can be left out. Surprisingly, it was determined that inventive formulations with PEG and the masking agent tasted better than formulations without PEG, with an equal amount of the masking agent.

### Preservative

Several preservatives were considered: methylparaben, propylparaben, butylparaben and sodium benzoate. Sodium benzoate has both bacteriostatic and antifungal properties attributed to undissociated benzoic acid, hence preservative efficacy is best seen in acidic solution (pH 2-5). In alkaline conditions it is almost without effect. Therefore, sodium benzoate is not suitable for NSG formula (pH around 7).

Guidelines IIG recommend the use of propylparaben at 0.01-0.02% levels in case when preservation required. Formulations with at least 25 % glycerin and propylene glycol are relatively anti-microbial and do not require additional preservation. A screen study according to USP Preservative effectiveness test was conducted using a series of formulations with varied levels of glycols.

### EXAMPLE 3

### Objective: Evaluate the effect of PEG-1000 level variation in the NSG Pseudoephedrine formulation.

Three 1.0 kg lab batches were compounded, whereby the level of the Polyethylene glycol 1000 (PEG 1000) was varied at 5 %, 10% and 15%.

In order to determine the effect of PEG 1000 a sensory evaluation test was conducted using six subjects. The subjects who evaluated two samples, the high (15%) level and the low (5 %) level, were asked to rate the intensity of sweetness, bitterness and flavor. The perceived intensities for sweetness and bitterness were rated on a 7-point scale. Respondents were trained in previous sessions to use the 7-point intensity scale as it applies to sweetness intensity. They were also asked to describe the texture of the two samples in their own words. Samples were evaluated in alternating sequence.

### RESULTS

Sweetness was perceived by four out of six subjects to be one unit higher for the 15% PEG sample, while two subjects rated the 5% PEG sample as one unit sweeter. Bitterness was rated one to two units lower for the 15% PEG sample by four subjects, while only one subject perceived the 5% PEG sample to be less bitter. This indicates that the higher PEG level tends to mask the unpleasantly bitter taste of the active ingredient and also increases the sweetness perception. The terms used to describe the texture for both samples were: "smooth", "jelly like", "syrupy" "oily", but there was no apparent distinction between the samples.

### CONCLUSION

Directionally the higher level of PEG 1000 was perceived as increasing sweetness and suppressing the bitterness perception.

### SENSORY TEST RESULTS

**TABLE 2**

| ***Subject*** | ***Evaluation sequence*** | ***Attribute intensity*** | ***Sample 1) 5%PEG1000 Batch S184-52981*** | ***Sample 2) 15% PEG 1000 Batch S184-52982*** |
|---|---|---|---|---|
| A. L. | Sample 2) | **Sweetness** | Slight to moderate (4) | Moderate (5) |
| | Sample 1) | ***Bitterness*** | Very slight (2) | Very slight (2) |
| | | ***Flavor*** | Slight to moderate | Moderate |
| | | ***Texture description*** | Syrupy, very smooth , compares to sample 2) | Syrupy, very smooth |
| S.A. | Sample 1) | ***Sweetness*** | Moderate to high (6) | High (7) |
| | Sample 2) | ***Bitterness*** | Threshold to slight (2) | Threshold (1) |
| | | ***Flavor*** | Moderate | Moderate |
| | | ***Texture description*** | Creamy, slightly oily, jelly like | Smooth, oily jelly like, compares to sample 1) |
| E.P. | Sample 2) | ***Sweetness*** | Moderate to high (6) | High (7) |
| | Sample 1) | ***Bitterness*** | Very slight to threshold (1) | None (0) |
| | | ***Flavor*** | Moderate to high | Moderate |
| | | ***Texture description*** | Smooth, syrupy | Smooth, jelly like, syrupy, smoother than sample 1 |
| E.Z. | Sample 1) | ***Sweetness*** | Slight (3) | Threshold to slight (2) |
| | Sample 2) | ***Bitterness*** | Moderate (5) | Slight (3) |
| | | ***Flavor*** | Moderate | Moderate |
| | | ***Texture description*** | Smooth, thick, creamy | Smooth, slightly "gummy", compares to sample 1 |
| P.v.H. | Sample 2) | ***Sweetness*** | Moderate + (5.5.) | Slight to moderate (4) |
| | Sample 1) | ***Bitterness*** | Slight to moderate (4) | Moderate to high (6) |
| | | ***Flavor*** | Moderate to high | Moderate |
| | | ***Texture description*** | Smooth, jelly like, compares to sample 1) | Smooth, jelly like |
| Z. B. | Sample 1) | ***Sweetness*** | Moderate to high (6) | High (7) |
| | Sample 2) | ***Bitterness*** | Threshold to slight (2) | Threshold (1) |
| | | ***Flavor*** | Moderate | Moderate |
| | | ***Texture description*** | Viscous gel, smooth, velvety mouthfeel | Viscous gel, smooth, slightly oily, waxy mouthfeel |

### Intensity scale:

**TABLE 3**

| Descriptive | Threshold | Threshold to slight | Slight | Slight to moderate | Moderate | Moderate to high | High |
|---|---|---|---|---|---|---|---|
| Numeric | 1 | 2 | 3 | 4 | 5 | 6 | 7 |

### TALLY OF INTENSITY SCORES

**TABLE 4**

| | Sample 1, 5% PEG 1000 | Sample 2 15 % PEG 1000 | Net Difference (Sample 2 minus sample 1) |
|---|---|---|---|
| Sweetness | 4, 6, 6, 3, 5.5, 6 | 5, 7, 7, 2, 4, 7 | 1, 1, 1, 1 |
| | Average: 6 | Average: 6 | -1, -1.5, |
| Bitterness | 2, 2, 1.5, 5, 4, 2 | 2, 1, 0, 3, 6, 1 | 0, -1, -1, -2, -1 |
| | Average: 4.4 | Average:2.1 | 2 |

### NSG PSE FORMULATION

**TABLE 5**

| | Batch S184-52981 | Batch S184-52982 |
|---|---|---|
| Water | 43.3 | 33.3 |
| Glycerin | 50.0 | 50.0 |
| Polyethylene glycol 1000 | 5.0 | 15.0 |
| Carbomer 934P | 0.59 | 0.59 |
| Grape bubble gum flavor | 0.15 | 0.15 |
| Sucralose liquid concentrate | 0.30 | 0.30 |
| Initial viscosity at 23°C, | cps 9490 | 10,090 |

| | | |
|---|---|---|
| pH was adjusted to within 5.5 to 7.0 with NaOH for this and the following formulations. | | |

### EXAMPLE 4

### Objective: Evaluate the effect of using PEG-1000 at 10% level in NSG Pseudoephedrine formulation as compared to alternate case of not using PEG 1000.

Two 1.0 kg lab batches were compounded, whereby Polyethylene glycol 1000 (PEG 1000) was used at 10% level in one and was replaced with the alternate substitute solvent propylene glycol in the second batch.

In order to determine the effect of PEG 1000 a sensory evaluation test was conducted using six subjects. The subjects who evaluated the two samples, were asked to rate the intensity of sweetness, bitterness and flavor. The perceived intensities for sweetness and bitterness were rated on a 7-point scale. Respondents were trained in previous sessions to use the 7-point intensity scale as it applies to sweetness intensity. They were also asked to describe the texture of the two samples in their own words. Samples were evaluated in alternating sequence.

### RESULTS

Sweetness was perceived higher by five of the six panelists for the sample that was formulated with 10% PEG 1000. Only one panelist perceived the sample with the propylene glycol as sweeter. The average sweetness score for the PEG sample was 5.7 as compared to 4.9. The bitterness of the PEG 1000 sample was found markedly lower by all, two panelists found no bitterness at all in this sample (score "0"). Average bitterness score is 1.8 for the PEG sample as compared to 3.6. The comments on texture reflect that all subjects noted a difference and perceived the sample with PEG 1000 smoother. The comments on flavor level indicate that the sample with PEG 1000 may require somewhat higher flavor level.

### CONCLUSION

It can be concluded that 10% PEG 1000 effected an increased sweetness and suppressed bitterness perception over a PEG-free control.

### SENSORY TEST RESULTS

**TABLE 6**

| ***Subject*** | ***Evaluation sequence*** | ***Attribute intensity*** | ***Sample 1) No PEG 1000 Batch S184-52925*** | ***Sample 2) 10% PEG 1000 Batch S184-52926*** |
|---|---|---|---|---|
| E. Z. | Sample 2) | **Sweetness** | Slight (3) | Slight to moderate (4) |
| | Sample 1) | ***Bitterness*** | Moderate (5) | Slight (3) |
| | | ***Flavor*** | Slight to moderate | Moderate |
| | | ***Texture description*** | Smooth, slightly less creamy than sample 2) | Syrupy, creamy, thicker than sample 1) |
| F.R. | Sample 1) | **Sweetness** | Moderate (5) | Moderate to high (6.5) |
| | Sample 2) | ***Bitterness*** | Threshold to slight (2) | None (0) |
| | | ***Flavor*** | Moderate, distinct grape | Slightly lower than 1) |
| | | ***Texture description*** | Smooth creamy, like honey | Smooth, thicker, more persistent smoothness |
| S.Ch. | Sample 2) | **Sweetness** | Moderate to high (6) | Moderate (5) |
| | Sample 1) | ***Bitterness*** | Moderate to high (6) | Slight to moderate (4) |
| | | ***Flavor*** | Slightly stronger than sample 2) | Slightly too strong |
| | | ***Texture description*** | Less smooth, thicker than sample 2) | Smooth |
| E.P. | Sample 1) | **Sweetness** | Moderate (5) | Moderate to high + (6.5) |
| | Sample 2) | ***Bitterness*** | Slight (3) | Threshold to plus (1.5) |
| | | ***Flavor*** | Strong, definite grape | Less, more candy type grape |
| | | ***Texture description*** | Smooth, jelly like | Smoother texture, spreads easily |
| B.W. | Sample 2) | **Sweetness** | Moderate plus (5.5.) | Moderate to high (6) |
| | Sample 1) | ***Bitterness*** | Slight plus (3.5) | Threshold to slight (2) |
| | | ***Flavor*** | Strong | Strong |
| | | ***Texture description*** | Less smooth than sample 1) less slippery | Smooth, syrupy |
| S.A. | Sample 1) | **Sweetness** | Moderate (5) | Moderate to high (6) |
| | Sample 2) | ***Bitterness*** | Threshold to slight (2) | None (0) |
| | | ***Flavor*** | Moderate | Moderate |
| | | ***Texture description*** | Smooth, palatable, somewhat "sticky" | Smooth, leaves clean palate |

### Intensity scale:

**TABLE 7**

| Descriptive | Threshold | Threshold to slight | Slight | Slight to moderate | Moderate | Moderate to high | High |
|---|---|---|---|---|---|---|---|
| Numeric | 1 | 2 | 3 | 4 | 5 | 6 | 7 |

### TALLY OF INTENSITY SCORES

**TABLE 8**

| | Sample 1, No PEG 1000 | Sample 2 10 % PEG 1000 | Net Difference (Sample 2 minus sample 1) |
|---|---|---|---|
| Sweetness | 3, 5, 6, 5, 5.5, 5 | 4, 6.5, 5, 6.5, 6, 6 | 1, 1.5, 1.5, 0.5, 1 |
| | Average: 4.9 | Average: 5.7 | -1, |
| Bitterness | 5, 2, 6, 3, 3.5, 2 | 3, 0, 4. 1.5, 2, 0 | -2, -2, -2, -1.5, -1.5, -2 |
| | Average: 3.6 | Average: 1.8 | |

### NSG PSE FORMULATION EXAMPLE

**TABLE 9**

| | Batch S184-52925 | Batch S184-52926 |
|---|---|---|
| Water | 38.5 | 38.5 |
| Glycerin | 50.0 | 50.0 |
| Propylene glycol | 10.0 | - |
| Polyethylene glycol 1000 | - | 10.0 |
| Carbomer 934P | 0.55 | 0.55 |
| Grape bubble gum flavor | 0.15 | 0.15 |
| Sucralose liquid concentrate | 0.30 | 0.30 |
| Initial viscosity at 23°C, cps | 6400 | 6730 |

### EXAMPLE 5

### Objective: Evaluate the taste effect of Acetaminophen NSG solution with PEG- 1000 as compared to alternate case of Acetaminophen NSG suspension without PEG 1000.

A 1.0 kg batch of acetaminophen NSG suspension was compound for the sensory evaluation. This batch contained no propylene glycol as and no PEG 1000. The other 10 kg batch was compounded for marketing study, and this batch contained 15% of PEG 1000 as a solvent of acetaminophen.

In order to determine the effect of PEG 1000, a sensory evaluation test was conducted using six subjects. The subjects, who evaluated the two samples, were asked to rate the intensity of sweetness, bitterness and flavor. The perceived intensities for sweetness and bitterness were rated on a 7-point scale. Respondents were trained in previous sessions to use the 7-point intensity scale as it applies to sweetness intensity. They were also asked to describe the texture of the two samples in their own words. Samples were evaluated in alternating sequence.

### RESULTS

Sweetness was perceived by three out of six subjects to be one to three units higher for the PEG 1000 formula. Three subjects failed to find the difference of the sweetness between two samples. Bitterness was rated half to three units lower for the PEG 1000 sample than that of the suspension sample by five out of six subjects, while only one subject perceived the suspension sample to be less bitter. This indicates that the PEG 1000 tends to mask the bitter taste of the acetaminophen and also increases the sweetness perception. It was also found that the PEG 1000 solution sample was smoother than the suspension sample.

### CONCLUSION

Acetaminophen NSG solution with 10% PEG 1000 is less bitter and smoother than the PEG-free suspension formula.

### SENSORY TEST RESULTS

**TABLE 10**

| ***Subject*** | ***Evaluation sequence*** | ***Attribute intensity*** | ***Sample 1) No PEG 1000 APAP suspension Batch S175-52303*** | ***Sample 2) 15% PEG 1000 APAP solution Batch S175-52970*** |
|---|---|---|---|---|
| E. P. | Sample 2) | **Sweetness** | Moderate to high (6) | Moderate to high (6) |
| | Sample 1) | ***Bitterness*** | Slight (3) | Threshold to slight +(2.5) |
| | | ***Flavor*** | Slightly less than sample 2 | Slightly weak grape flavor |
| | | ***Texture description*** | Slightly less smooth than sample 2 (sl. coarse) | Smooth |
| X.W. | Sample 1) | **Sweetness** | Moderate (5) | Moderate to strong (6) |
| | Sample 2) | ***Bitterness*** | Slight (3) | Threshold plus (1.5) |
| | | ***Flavor*** | Moderate | Moderate |
| | | ***Texture description*** | Less smooth than sample 2 | Smooth (slightly smoother than sample 1) |
| Z.B. | Sample 2) | **Sweetness** | Moderate (5) | Moderate to high (6) |
| | Sample 1) | ***Bitterness*** | Slight + (3.5) | Threshold plus (1.5) |
| | | ***Flavor*** | Slight to moderate | Moderate |
| | | ***Texture description*** | Smooth initially than coarse, slightly gritty | Very smooth, creamy |
| E.Z. | Sample 1) | **Sweetness** | Slight (3) | Moderate (6) |
| | Sample 2) | ***Bitterness*** | Moderate plus (5.5) | Slight to moderate (4) |
| | | ***Flavor*** | Moderate | Moderate |
| | | ***Texture description*** | Smooth, slightly coarse, thick | Smooth, thick, pudding like |
| S | Sample 2) | **Sweetness** | Moderate to high (6) | Moderate to high (6) |
| | Sample 1) | ***Bitterness*** | Moderate (5) | Threshold (1) |
| | | ***Flavor*** | Slightly to strong | Moderate |
| | | ***Texture description*** | Creamy, thick | Smooth, thick |
| S.A. | Sample 1) | **Sweetness** | Moderate to high (6) | Moderate to high (6) |
| | Sample 2) | ***Bitterness*** | Slightly (3) | Slight to moderate (4) |
| | | ***Flavor*** | Moderate | Moderate plus |
| | | ***Texture description*** | Creamy | Smooth |

### Intensity scale:

**TABLE 11**

| Descriptive | Threshold | Threshold to slight | Slight | Slight to moderate | Moderate | Moderate to high | High |
|---|---|---|---|---|---|---|---|
| Numeric | 1 | 2 | 3 | 4 | 5 | 6 | 7 |

### TALLY OF INTENSITY SCORES

**TABLE 12**

| | Sample 1, No PEG 1000 | Sample 2 10 % PEG 1000 | Net Difference (Sample 2 minus sample 1) |
|---|---|---|---|
| Sweetness | Average: 5.2 | Average: 6.0 | 0.8 |
| Bitterness | Average: 3.8 | Average: 2.4 | -1.6 |

### NSG APAP FORMULATION EXAMPLE

**TABLE 13**

| | Batch S175-53004 | Batch S175-52970 |
|---|---|---|
| Water | 55.73% | 30.93% |
| Glycerin | 35% | 50% |
| Propylene glycol | N/A | N/A |
| Polyethylene glycol 1000 | N/A | 15% |
| Sorbital crystalline | 5% | N/A |
| Sucralose liquid concentrate | 0.4% | 0.4% |
| Carbomer 934P | 0.28% | 0.29% |
| Grape flavor | 0.15% | 0.15% |
| Masking Agent | 0.4% | 0.4% |

### EXAMPLE 6

### Objective: Evaluate the effect of using PEG 1000 in NSG Acetaminophen formulation as compared to alternate case of using propylene glycol.

A 1.0 kg batch was compound for the sensory evaluation. This batch contained 25% of propylene glycol as a solvent and no PEG 1000. The other 10 kg batch contained 15% of PEG 1000 as a solvent of acetaminophen.

In order to determine the effect of PEG 1000, a sensory evaluation test was conducted using six subjects. The subjects who evaluated the two samples, were asked to rate the intensity of sweetness, bitterness and flavor. The perceived intensities for sweetness and bitterness were rated on a 7-point scale. Respondents were trained in previous sessions to use the 7-point intensity scale as it applies to sweetness intensity. They were also asked to describe the texture of the two samples in their own words. Samples were evaluated in alternating sequence.

### RESULTS

Sweetness was perceived by five out of six subjects to be at least one unit higher for the PEG 1000 sample than that of the propylene glycol sample, while one subject failed to find the difference of the sweetness between two samples. Bitterness was rated two to four units lower for the PEG 1000 sample than the propylene glycol samples by all of the subjects. This indicates that the higher PEG 1000 level tends to mask the bitter taste of the acetaminophen and also increases the sweetness perception. The terms used to describe the texture for both samples were: "smooth", "honey like", "sticky", but there was no apparent distinction between the samples.

### CONCLUSION

PEG 1000 increases the sweetness and masks the bitterness perception of the acetaminophen NSG formula.

### SENSORY TEST RESULTS

**TABLE 14**

| ***Subject*** | ***Evaluation sequence*** | ***Attribute intensity*** | ***Sample 1) 25% Propylene Glycol APAP solution Batch S175-52303*** | ***Sample 2) 15% PEG 1000 APAP solution Batch S175-52970*** |
|---|---|---|---|---|
| X.W. | Sample 1) | **Sweetness** | Moderate (5) | Moderate to high (6) |
| | Sample 2) | ***Bitterness*** | Slight to Moderate (4) | Threshold to slight (2) |
| | | ***Flavor*** | Moderate | Moderate |
| | | ***Texture description*** | Smooth | Smooth |
| Z.B. | Sample 2) | **Sweetness** | Moderate to high (6) | Moderate to high (6) |
| | Sample 1) | ***Bitterness*** | Slight to moderate (3.75), after taste | Threshold (1) |
| | | ***Flavor*** | Slightly less than Moderate | Moderate |
| | | ***Texture description*** | Thinner, smooth | Smooth, thicker (more body) |
| E.P. | Sample 1) | **Sweetness** | (2.5) | Moderate (5) |
| | Sample 2) | ***Bitterness*** | Moderate to high (6), after taste | Slight (3) |
| | | ***Flavor*** | Slight to moderate | Less intensity than the other sample |
| | | ***Texture description*** | Smooth | Smooth |
| B.W. | Sample 2) | **Sweetness** | Slight to Moderate (4.5) | Moderate to high (6) |
| | Sample 1) | ***Bitterness*** | Moderate to high (6), after taste | Slight to moderate (3.5) |
| | | ***Flavor*** | Less than Moderate | Moderate |
| | | ***Texture description*** | Smooth, no sticky | Sticky, honey like |
| S.A. | Sample 1) | **Sweetness** | Moderate (5) | Moderate to high (6) |
| | Sample 2) | ***Bitterness*** | Moderate (5) | Threshold to slight (2) |
| | | ***Flavor*** | Less than moderate | Less than moderate |
| | | ***Texture description*** | Smooth | Smooth |
| P.H. | Sample 2) | **Sweetness** | Slightly (3) | Slight to Moderate (4) |
| | Sample 1) | ***Bitterness*** | High (7), after taste | Slight (3) |
| | | ***Flavor*** | Slight | Moderate to high |
| | | ***Texture description*** | Smooth | Smooth |

### Intensity scale

**TABLE 15**

| Descriptive | Threshold | Threshold to slight | Slight | Slight to moderate | Moderate | Moderate to high | High |
|---|---|---|---|---|---|---|---|
| Numeric | 1 | 2 | 3 | 4 | 5 | 6 | 7 |

### TALLY OF INTENSITY SCORES

**TABLE 16**

| | Sample 1, No PEG 1000 | Sample 2 10 % PEG 1000 | Net Difference (Sample 2 minus sample 1) |
|---|---|---|---|
| Sweetness | Average: 4.3 | Average: 5.5 | 1.2 |
| Bitterness | Average: 5.3 | Average: 2.9 | -2.4 |

### NSG APAP FORMULATION EXAMPLE

**TABLE 17**

| | Batch S175-53003 | Batch S175-52970 |
|---|---|---|
| Water | 26.12% | 30.93% |
| Glycerin | 40% | 50% |
| Propylene glycol | 25% | N/A |
| Polyethylene glycol 1000 | N/A | 15% |
| Sorbital crystalline | 5% | N/A |
| Sucralose liquid concentrate | 0.4% | 0.4% |
| Carbomer 934P | 0.265% | 0.29% |
| Grape flavor | 0.15% | 0.15% |
| Masking Agent | 0.2% | 0.4% |

### REFERENCES

1. Martindale's The Extra Pharmacopoeia, 29th Ed., J.E. Reynolds (Ed.), London, The Pharmaceutical Press, 1989, Pg 908.
2. Remington's Pharm. Sciences 18th Ed., A.R. Gennaro (Ed.)., Mack Publishing Co., Easton Penn., Pg. 865.
3. United States Pharmacopoeia 24,U.S. Pharm. Convention Inc., Rockville, MD., Mack Printing Co., Easton, PA.
4. Pharmaceutical Dosage Forms, Volume 1, Marcel Dekker, Inc., New York and Basel.

## Claims

1. A spill resistant pharmaceutical formulation for oral administration, comprising a pharmaceutical agent, a carbomer and a bitterness masking amount of from about 1% to about 25% wt/wt low-weight polyethylene glycol (PEG).

2. The formulation of claim 1, which tastes less bitter and more sweet in a taste test than an equivalent formulation having water or propylene glycol substituted for the PEG.

3. The formulation of claim 1, wherein the active ingredient is pseudoephedrine.

4. The formulation of claim 1, wherein the active ingredient is acetaminophen.

5. The formulation of claim 1, wherein the formulation is a suspension.

6. The formulation of claim 1, wherein the formulation is a solution.

7. The formulation of claim 1, wherein the low-weight PEG concentration is in the range from about 5 to about 15%.

8. The formulation of claim 1, wherein the low-weight PEG is PEG 600 to PEG 1500.

9. The formulation of claim 1, wherein the low-weight PEG is selected from the group consisting of PEG 200, PEG 300, PEG 400, PEG 540, PEG 600, PEG 800, PEG 900, PEG 1000, PEG 1450, PEG 1540 and combinations.

10. The formulation of claim 1, wherein the low-weight PEG is PEG 600 or PEG 1000 or combinations.

11. The formulation of claim 1, comprising about 5% to about 15% PEG, up to about 30 or 45 % water, up to about 40 to 50% glycerin, up to 10 % active ingredient, up to about 0.2% to about 0.6% sucralose liquid concentrate, about 0.2 to about 1.0 % Carbomer 934P, and a selected commercially acceptable flavoring agent.

12. The formulation of claim 1, comprising from at least about 1% to about 20% polyethylene glycol (PEG), up to about 45 % water, up to about 50% active ingredient, up to about 0.4% sucralose liquid concentrate, up to about 0.59% Carbomer 934P, and up to about 0.15% grape flavoring agent.

13. The formulation of claim 1, comprising about 30% water, about 50% glycerin, about 15% polyethylene glycol 1000, about 0.4% sucralose liquid concentrate, about 0.29% Carbomer 934P, and about 0.15% grape flavoring agent.

14. The formulation of claim 1, comprising about 38% water, about 50.0% glycerin, about 10.0% polyethylene glycol 1000, about 0.55% Carbomer 934P, about 0.15% grape bubble gum flavoring agent, and about 0.30% sucralose liquid concentrate.

15. The formulation of claim 1, comprising about 33% water, about 50.0% glycerin, about 15.0% polyethylene glycol 1000, about 0.59% Carbomer 934P, about 0.15% grape bubble gum flavoring agent, and about 0.30% sucralose liquid concentrate.

16. The formulation of claim 1, comprising about 43% water, about 50.0% glycerin, about 5.0% polyethylene glycol 1000, about 0.59% Carbomer 934P, about 0.15% flavoring agent, and about 0.30% sucralose liquid concentrate.

17. The formulation of claim 1, wherein the spill resistant pharmaceutical composition does not contain a seaweed polysaccharide selected from the group consisting of agar, algin, carrageenan and furcelleran or a mixture thereof.

18. The formulation of claim 1, wherein the spill resistant pharmaceutical composition has antibacterial and antifungal properties without an added preservative.

19. The formulation of claim 1, having a viscosity of above 2500 cps.

20. The formulation of claim 19, wherein the viscosity is between about 5000 and about 15, 000 cps.

21. The formulation of claim 19, wherein the viscosity is between about 2500 cps and about 25000 cps.

22. The formulation of claim 1, wherein the low-weight PEG concentration is below 20%.

23. The formulation of claim 1, wherein the low-weight PEG concentration is below 15%.

24. A method of making a semi-solid formulation, comprising
(a) determining a bitterness masking amount of a low-weight polyethylene glycol (PEG), and
(b) adding said bitterness masking amount of PEG to a pharmaceutical agent and carbomer to form a spill resistant pharmaceutical formulation for oral administration as set forth in claim 1,
wherein the bitterness masking amount of low-weight PEG is from about 1% to about 25%.

## Patentansprüche

1. Eine verschüttungssichere pharmazeutische Formulierung zur oralen Verabreichung umfassend einen pharmazeutischen Wirkstoff, ein Carbomer und eine die Bitterkeit maskierende Menge von etwa 1 % bis etwa 25% wt/wt nieder-gewichtiges Polyethylenglykol (PEG).

2. Die Formulierung nach Anspruch 1, die weniger bitter und süßer schmeckt in einem Geschmackstest als eine äquivalente Formulierung, die PEG durch Wasser oder Propylenglykol ersetzt hat.

3. Die Formulierung nach Anspruch 1, wobei der Wirkstoff Pseudoephedrin ist.

4. Die Formulierung nach Anspruch 1, wobei der Wirkstoff Acetaminophen ist.

5. Die Formulierung nach Anspruch 1, wobei die Formulierung eine Suspension ist.

6. Die Formulierung nach Anspruch 1, wobei die Formulierung eine Lösung ist.

7. Die Formulierung nach Anspruch 1, wobei die nieder-gewichtige PEG-Konzentration im Bereich von etwa 5 bis etwa 15 % ist.

8. Die Formulierung nach Anspruch 1, wobei das nieder-gewichtige PEG PEG 600 bis PEG 1500 ist.

9. Die Formulierung nach Anspruch 1, wobei das nieder-gewichtige PEG ausgewählt ist aus der Gruppe bestehend aus PEG 200, PEG 300, PEG 400, PEG 540, PEG 600, PEG 800, PEG 900, PEG 1000, PEG 1450, PEG 1540 und Kombinationen.

10. Die Formulierung nach Anspruch 1, wobei das nieder-gewichtige PEG PEG 600 oder PEG 1000 oder Kombinationen ist.

11. Die Formulierung nach Anspruch 1, umfassend etwa 5 % bis etwa 15 % PEG, bis zu etwa 30 oder 45 % Wasser, bis zu etwa 40 bis 50 % Glycerin, bis zu 10 % Wirkstoff, bis zu etwa 0,2 % bis etwa 0,6 % Sucralose-Flüssigkonzentrat, etwa 0,2 % bis 1,0 % Carbomer 934P und ein ausgewählter handelsüblicher Aromastoff.

12. Die Formulierung nach Anspruch 1, umfassend mindestens etwa 1 % bis etwa 20 % Polyethylenglykol (PEG), bis zu etwa 45 % Wasser, bis zu etwa 50 % Wirkstoff, bis zu etwa 0,4 % Sucralose-Flüssigkonzentrat, bis zu etwa 0,59 % Carbomer 934P und bis zu etwa 0,15 % Trauben-Aromastoff.

13. Die Formulierung nach Anspruch 1, umfassend etwa 30 % Wasser, etwa 50 % Glycerin, etwa 15 % Polyethylenglykol 1000, etwa 0,4 % Sucralose-Flüssigkonzentrat, etwa 0,29 % Carbomer 934P und etwa 0,15 % Trauben-Aromastoff.

14. Die Formulierung nach Anspruch 1, umfassend etwa 38 % Wasser, etwa 50,0 % Glycerin, etwa 10,0 % Polyethylenglykol 1000, etwa 0,55 % Carbomer 934P, etwa 0,15 % Trauben-Kaugummi-Aromastoff und etwa 0,30 % Sucralose-Flüssigkonzentrat.

15. Die Formulierung nach Anspruch 1, umfassend etwa 33 % Wasser, etwa 50,0 % Glycerin, etwa 15,0 % Polyethylenglykol 1000, etwa 0,59 % Carbomer 934P, etwa 0,15 % Trauben-Kaugummi-Aromastoff und etwa 0,30 % Sucralose-Flüssigkonzentrat.

16. Die Formulierung nach Anspruch 1, umfassend etwa 43 % Wasser, etwa 50,0 % Glycerin, etwa 5,0 % Polyethylenglykol 1000, etwa 0,59 % Carbomer 934P, etwa 0,15 % Aromastoff und etwa 0,30 % Sucralose-Flüssigkonzentrat.

17. Die Formulierung nach Anspruch 1, wobei die verschüttungssichere pharmazeutische Zusammensetzung kein Algen-Polysaccharid ausgewählt aus der Gruppe bestehend aus Agar, Algin, Carrageenan und Furcellaran oder ein Gemisch davon, enthält.

18. Die Formulierung nach Anspruch 1, wobei die verschüttungssichere pharmazeutische Zusammensetzung antimikrobielle und antimykotische Eigenschaften ohne ein hinzugefügtes Konservierungsmittel hat.

19. Die Formulierung nach Anspruch 1, die eine Viskosität von etwa 2500 cps hat.

20. Die Formulierung nach Anspruch 19, wobei die Viskosität zwischen etwa 5000 und etwa 15000 cps ist.

21. Die Formulierung nach Anspruch 19, wobei die Viskosität zwischen etwa 2500 und etwa 25000 cps ist.

22. Die Formulierung nach Anspruch 1, wobei die nieder-gewichtige PEG-Konzentration unter 20 % ist.

23. Die Formulierung nach Anspruch 1, wobei die nieder-gewichtige PEG-Konzentration unter 15 % ist.

24. Ein Verfahren zur Zubereitung einer halb-festen Formulierung, umfassend
a) Bestimmung einer die Bitterkeit maskierenden Menge eines nieder-gewichtigen Polyethylenglykols (PEG), und
b) Zugabe der die Bitterkeit maskierende Menge von PEG zu einem pharmazeutischen Wirkstoff und Carbomer, um eine verschüttungssichere pharmazeutische Formulierung zur oralen Verabreichung zu bilden, wie in Anspruch 1 dargelegt, wobei die die Bitterkeit maskierende Menge von nieder-gewichtigem PEG etwa 1 % bis etwa 25 % ist.

## Revendications

1. Formulation pharmaceutique pour une administration orale résistante à l'écoulement, comprenant un agent pharmaceutique, un carbomère et une quantité pour masquer l'amertume de polyéthylène glycol (PEG) de bas poids d'environ 1 % à 25% en poids.

2. Formulation selon la revendication 1, ayant un goût moins amer et plus sucré dans un test de goût qu'une formulation équivalente comprenant de l'eau ou du polypropylène glycol à la place du PEG.

3. Formulation selon la revendication 1, dans laquelle l'ingrédient actif est la pseudoéphédrine.

4. Formulation selon la revendication 1, dans laquelle l'ingrédient actif est l' acétaminophène.

5. Formulation selon la revendication 1, dans laquelle la formulation est une suspension.

6. Formulation selon la revendication 1, dans laquelle la formulation est une solution.

7. Formulation selon la revendication 1, dans laquelle la concentration du PEG de bas poids est d'environ 5 à environ 15%.

8. Formulation selon la revendication 1, dans laquelle le PEG de bas poids est le PEG 600 à 1500.

9. Formulation selon la revendication 1, dans laquelle le PEG de bas poids est sélectionné dans le groupe consistant en le PEG 200, le PEG 300, le PEG 400, le PEG 540, le PEG 600, le PEG 800, le PEG 900, le PEG 1000, le PEG 1450, le PEG 1540 et leurs combinaisons.

10. Formulation selon la revendication 1, dans laquelle le PEG de bas poids est le PEG 600 ou le PEG 1000 ou leurs combinaisons.

11. Formulation selon la revendication 1, comprenant d'environ 5% à environ 15% de PEG, jusqu'à environ 30 ou 45% d'eau, jusqu'à environ 40 à 50% de glycérine, jusqu'à environ 10% d'ingrédient actif, jusqu'à environ 0,2 à environ 0,6% de concentré liquide de sucralose, environ 0,2 jusqu'à environ 1,0% de Carbomer 934P, et un exhausteur de goût sélectionné commercialement acceptable.

12. Formulation selon la revendication 1, comprenant environ 1% à environ 20% de polyéthylène glycol (PEG), jusqu'à environ 45% d'eau, jusqu'à environ 50% d'ingrédient actif, jusqu'à environ 0,4% de concentré liquide de sucralose, jusqu'à environ 0,59% de Carbomer 934P, et jusqu'à environ 0,15% d'exhausteur de goût de raisin.

13. Formulation selon la revendication 1, comprenant environ 30% d'eau, environ 50% de glycérine, environ 15% de PEG 1000, environ 0,4% de concentré liquide de sucralose, environ 0,29% de Carbomer 934P, et environ 0,15% d'exhausteur de goût de raisin.

14. Formulation selon la revendication 1, comprenant environ 38% d'eau, environ 50,0% de glycérine, environ 10,0% de PEG 1000, environ 0,55% de Carbomer 934P, environ 0,15% d'exhausteur de goût à base de gomme de raisin, et environ 0,30% de concentré liquide de sucralose.

15. Formulation selon la revendication 1, comprenant environ 33% d'eau, environ 50,0% de glycérine, environ 15% de PEG 1000, environ 0,59% de Carbomer 934P, environ 0,15% d'exhausteur de goût à base de gomme de raisin, et environ 0,30% de concentré liquide de sucralose.

16. Formulation selon la revendication 1, comprenant environ 43% d'eau, environ 50,0% de glycérine, environ 5,0% de PEG 1000, environ 0,59% de Carbomer 934P, environ 0,15% d'exhausteur de goût, et environ 0,30% de concentré liquide de sucralose.

17. Formulation selon la revendication 1, dans laquelle la composition résistante à l'écoulement ne contient pas un polysaccharide d'origine de varech sélectionné dans le groupe consistant en l'agar-agar, l'algin, le carraghénane et le furcellerane ou une combinaison desdits composés.

18. Formulation selon la revendication 1, dans laquelle la composition résistante à l'écoulement possède les propriétés antimycosiques et antibactériennes sans ajout de conservateur.

19. Formulation selon la revendication 1 ayant une viscosité au-dessus de 2500 cps.

20. Formulation selon la revendication 19, dans laquelle la viscosité est entre environ 5000 et environ 15000 cps.

21. Formulation selon la revendication 19, dans laquelle la viscosité est entre environ 2500 et environ 25000 cps.

22. Formulation selon la revendication 1, dans laquelle la concentration en PEG de bas poids est en-dessous de 20%.

23. Formulation selon la revendication 1, dans laquelle la concentration en PEG de bas poids est en-dessous de 15%.

24. Méthode pour fabriquer une formulation semi-solide, comprenant
a. la détermination d'une quantité pour masquer l'amertume de polyéthylène glycol (PEG) de bas poids, et
b. l'ajout d'une quantité pour masquer l'amertume de PEG à un agent pharmaceutique et un carbomère pour former une formulation résistante à l'écoulement pour une administration orale comme décrit dans la revendication 1,
dans laquelle la quantité pour masquer l'amertume du PEG de bas poids est d'environ 1 % jusqu'à environ 25%.
